(19) Europäisches Patentamt
European Patent Office
Office européen des brevets

(11) **EP 2 709 685 B1**

(12) **EUROPEAN PATENT SPECIFICATION**

(45) Date of publication and mention
of the grant of the patent:
**11.05.2016 Bulletin 2016/19**

(21) Application number: **12721937.6**

(22) Date of filing: **16.05.2012**

(51) Int Cl.:
***A61M 1/00*** *(2006.01)* ***A61F 11/00*** *(2006.01)*

(86) International application number:
**PCT/IE2012/000022**

(87) International publication number:
**WO 2012/156959 (22.11.2012 Gazette 2012/47)**

(54) **A FLUID REMOVAL AND DELIVERY APPARATUS**

FLÜSSIGKEITENTFERNUNGS- UND AUSGABEVORRICHTUNG

APPAREIL DE RETRAIT ET DE DISTRIBUTION DE FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2011 US 201161486344 P**
**27.01.2012 US 201261591390 P**

(43) Date of publication of application:
**26.03.2014 Bulletin 2014/13**

(73) Proprietor: **Cork Institute Of Technology**
**County Cork (IE)**

(72) Inventors:
• **VAUGHAN, John**
**Cork (IE)**
• **O'DRISCOLL, Olive**
**Kinsale**
**County Cork (IE)**

• **O'CONNOR, Patrick**
**Mallow**
**County Cork (IE)**

(74) Representative: **Weldon, Michael James et al**
**John A. O'Brien & Associates**
**Third Floor,**
**Duncairn House,**
**14 Carysfort Avenue**
**Blackrock, Co. Dublin (IE)**

(56) References cited:
| EP-A1- 1 389 458 | WO-A2-2005/115527 |
| US-A- 4 702 733 | US-A- 5 484 402 |
| US-A- 5 902 264 | US-A- 5 916 150 |
| US-A- 6 024 726 | US-A1- 2007 244 425 |

## Description

Introduction

The invention relates to an apparatus for removal and delivery of a fluid such as a drug to the human or animal body.

A particular problem arises for delivery of drugs to sensitive and small-scale parts of the body, such as the ear. An example is treatment of Otitis Media ("OM"), an inflammation or infection of the middle ear in humans or animals. Traditionally, treatment has involved administration of oral antibiotics and/or insertion of tymponastomy tubes ("grommets"). Administration of oral antibiotics is a blunt approach giving rise to side effects. Therefore it has been proposed to deliver drugs directly to the affected area.

US6024726 describes an aspirator for the middle ear, which has a needle which penetrates the tympanic membrane. US5664094 also describes an aspirator for collecting middle ear specimens. It has a bulb to draw the specimen through a needle when the needle has punctured the ear drum. US6390975 describes a speculum for use in otoscopy, particularly for guiding a needle for delivery of a medicament. WO2005/11552 describes an apparatus for combined aspiration and medicament delivery. It has a trigger mechanism for spring-loaded actions of a pre-determined nature, in which frangible seals are broken. Pressing of buttons indexes a spring loaded turret barrel through pre-set positions, the trigger press giving a pre-set action in terms of quantity and duration.

The invention is directed towards providing an apparatus for improved fluid removal and drug delivery for such applications. One object is to achieve improved use control both of handling of the apparatus and of the extent of vacuum applied and medicament delivered.

US5902264 (Toso et al) describes an endoscopic surgical instrument for aspiration and irrigation. EP 1389458 (Nagashima Medical) describes a medical rinsing and sucking device.

Summary of the Invention

According to the invention, there is provided an apparatus for removal and delivery of fluids to a human or animal body, the apparatus having the features set out in claim 1.

In one embodiment, the fluid supply and the vacuum device extend from a distal end of the housing. In one embodiment, the fluid supply includes a plunger in a reservoir having an outlet linked with the valve. In one embodiment, the vacuum device comprises a sealed vacuum chamber which may be pierced. Preferably, the valves are linked in a Y configuration with a single outlet to the lumen.

In one embodiment, at least one valve is adapted to allow intermediate positions between fully open and fully closed, and a default closed position. In one embodiment, there is infinite variability between said open and closed positions.

In one embodiment, at least one valve is adapted to remain open for a variable duration according to user operation of the actuator. In one embodiment, at least one valve includes a spring-loaded pinching member to pinch a flexible tube. In one embodiment, the pinching member is biased to press the flexible conduit against a static part of the apparatus. Preferably, at least one pinching member is integral with a user actuator.

In one embodiment, the apparatus further comprises a safety feature which prevents fluid delivery before application of a vacuum. In one embodiment, said safety feature comprises a break-away tab or pivoting pawl which engages sloping teeth.

In one embodiment, said finger is the index finger. In one embodiment, one actuator comprises a push button and the other actuator comprises a lever.

In one embodiment, the extension mechanism comprises a spring-biased carriage arranged to slide under user control with an actuator to push the lumen, and to allow it to retract.

In one embodiment, the carriage is adapted to automatically retract under spring bias to retract the lumen. In one embodiment, the extension mechanism actuator is adapted to be operated by a user's second finger. In one embodiment, the extension mechanism actuator comprises a lever which pivots a cam.

In one embodiment, the extension mechanism actuator comprises a rotatable member threadably engaged with the housing to move axially upon user rotation.

In one embodiment, the lumen is adapted to bend or deform if an obstruction is encountered which presents a resistance greater than a threshold. In one embodiment, the lumen is adapted to deform or bend if an obstruction is encountered in the ear area which is not the tympanic membrane. In one embodiment, n the lumen has a flexibility causing it to deform by buckling. In one embodiment, the lumen has a tip which is configured to deform. Preferably, the tip has a concave surface. In one embodiment, the tip has a conical shape. In one embodiment, the tip is solid, without a channel, and a lumen channel terminates in one or more apertures near the tip. In one embodiment, the lumen tip narrows with a bevelled concave surface on one side.

In one embodiment, the lumen includes graduation marking to for visual guidance during lumen extension.

In one embodiment, the extension mechanism comprises a lever actuator for causing movement of the lumen. In one embodiment, the actuator is adapted to yield if excessive force is applied to move a lumen into an obstruction. In one embodiment, the actuator is adapted to crack at an engineered weak point.

In one embodiment, the apparatus comprises a fixture to connect with a visualisation device such as an otoscope. In one embodiment, the fixture comprises clips for snap-fitting connection of a visualisation device. Preferably, the fixtures are arranged to retain the visualisation device in an orientation parallel to the housing.

In one embodiment, the apparatus further comprises a speculum linked by a sleeve to the housing, the lumen extending through the sleeve.

In one embodiment, the speculum is adapted to be connected to a visualisation device such as an otoscope.

In one embodiment, the speculum comprises a channel to receive and retain the sleeve.

In one embodiment, the speculum is configured to assist location of the lumen for a desired application of the apparatus

Detailed Description of the Invention

The invention will be more clearly understood from the following description of some embodiments thereof, given by way of example only with reference to the accompanying drawings in which:-

Fig. 1 is a block diagram indicating the main parts of a fluid removal and delivery apparatus of the invention;

Fig. 2 is a perspective view of the apparatus in use, Fig. 3 is an exploded view, and Figs. 4 and 5 are cross-sectional views showing the apparatus in use;

Fig. 6 shows a needle tip having a concave conical shape for penetration of the tympanic membrane; and Fig. 7 shows an alternative configuration, in this case having a concave bevelled or tapered shape;

Figs. 8 shows a cam mechanism of the apparatus in more detail, with failure if excessive force is used;

Fig. 9 is a perspective view of an alternative apparatus, Fig. 10 sows the apparatus in use, and Fig. 11 shows attachment of an otoscope,

Fig. 12 is a cut-away view showing the internal mechanisms in more detail, and Figs. 13 and 14 are enlarged views illustrating how a needle is moved and yields if an obstruction is encountered; and

Fig. 15 is a view showing buckling of the lumen tip if an obstruction is encountered.

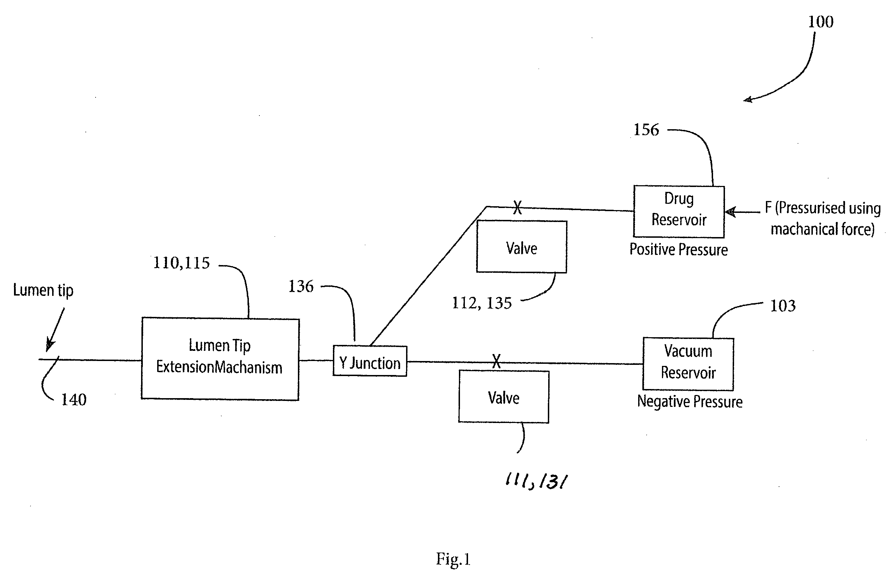

Referring to Fig. 1 a fluid withdrawal and delivery apparatus 100 has a medicament or drug reservoir 156 at the outlet of which there is a valve 112, 135, and a vacuum reservoir 103 at the outlet of which there is a valve 111, 131. Both valves are connected via a Y-junction 136 to a lumen tip extension mechanism 110, 115 for a needle or lumen 140.

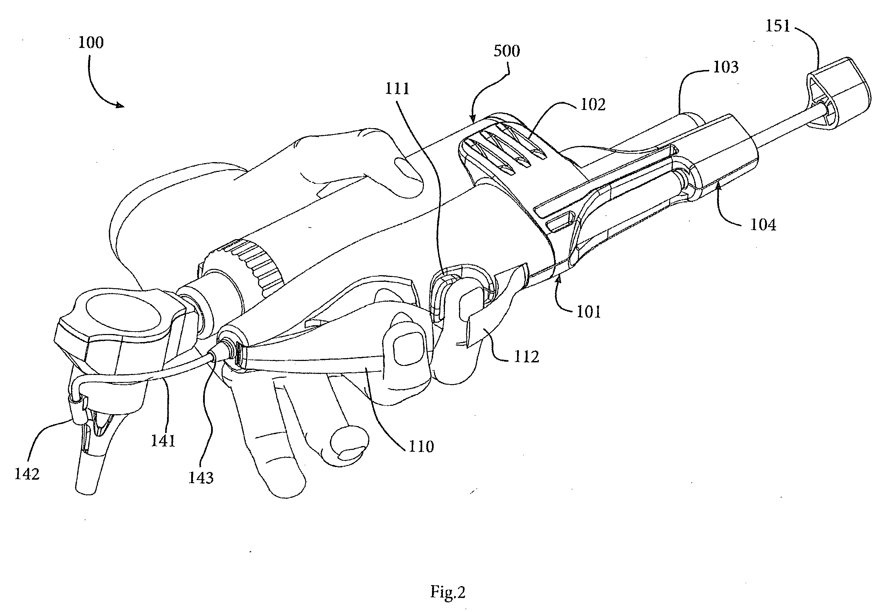

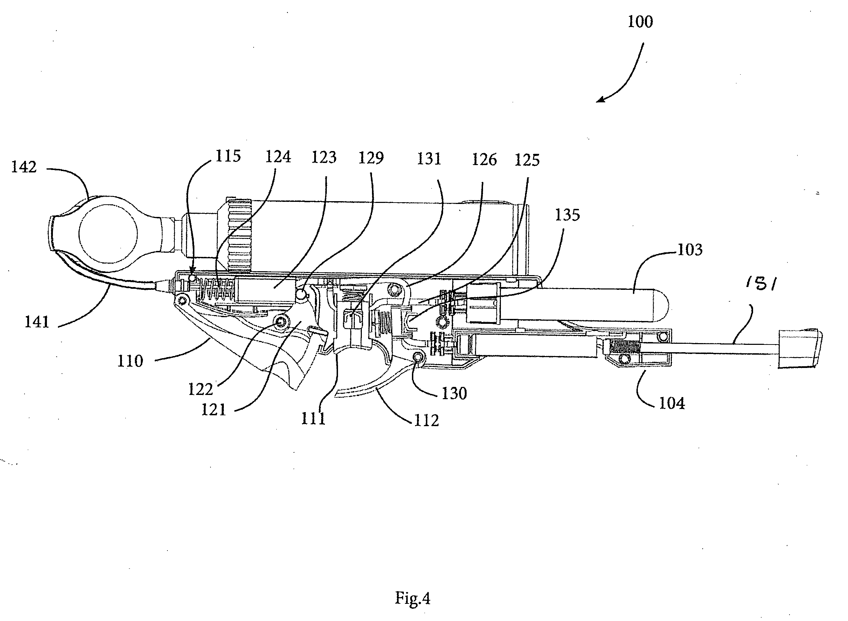

Referring to Figs. 2 to 5, the apparatus 100 comprises a housing 101 with a pair of opposed clamps 102 for gripping an otoscope 500, which itself does not form part of the invention. The lumen 140 extends through a sleeve 141 and the end of the sleeve 141 is mounted to a speculum 142, which in use is attached to the otoscope 500 as shown in Fig. 2. The vacuum device 103 and the drug supply device 104 extend from the rear of the apparatus 100. The drug supply device 104 has a plunger 151.

As shown most clearly in Fig. 2, there is a lever 110 for the second finger to cause the lumen 140 to project out from a safe position in the speculum, a push button 111 for the index finger to cause application of a vacuum, and a lever 112 for operation also by the same index finger to cause drug delivery. The apparatus 100 may therefore be used with only one hand, which is particularly advantageous as it allows the clinician to pull up and back on the pinna to facilitate straightening of the ear canal for visualisation of the tympanic membrane..

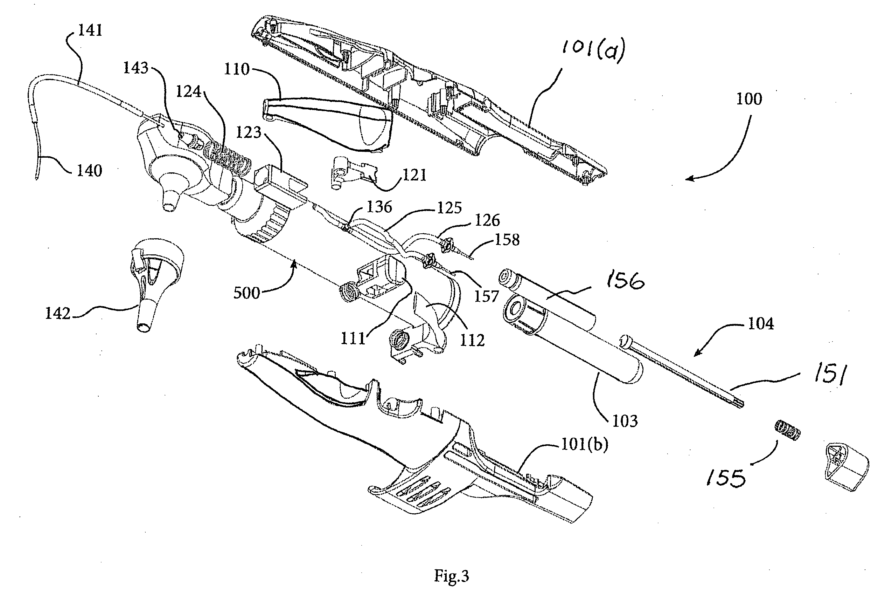

As shown most clearly in Figs. 3 to 5, the lumen extension mechanism lever 110 is on a rotating cam 121 pivoting about a pivot joint 122, so that a cam surface 129 pushes against a carriage 123 of an extension mechanism 115 for the lumen 140. This pushing action is against the force of a spring 124 within the mechanism 115. As is described in more detail below, the lumen 140 is adapted to yield by bending or deforming so that it does not protrude through skin or bone, only the ear drum.

The push button 111 is spring biased to pinch a vacuum tube 125 against a V-shaped feature 131 which is integral with the housing 101. The button 111 therefore pinches the vacuum tube 125 by default to provide a default valve closed position. Also, because the action is pinching the user can control flow through the tube 125 between the extremes of fully open and fully closed, and moreover the user has complete control over the duration of any position.

The lever 112 is pivoted about a joint 130 with spring bias against a V-shaped feature 135 which is integral with the housing 101. This action pinches a drug delivery tube 126 by default. Again, the user has complete control over the extent of opening and closing and of the durations, as for the vacuum application. Because the valve closing action is pressing against a flexible tube for both vacuum application and drug deliver, there is infinite control between the extreme open and closed positions. Hence the clinician has complete control over volume and duration of these operations.

The tubes 125 and 126 are interconnected at a junction 136 which has a single link to the carriage 123 so that they communicate with the lumen 140 in use. The carriage 123 is biased against the cam 129 by a helical spring 124.

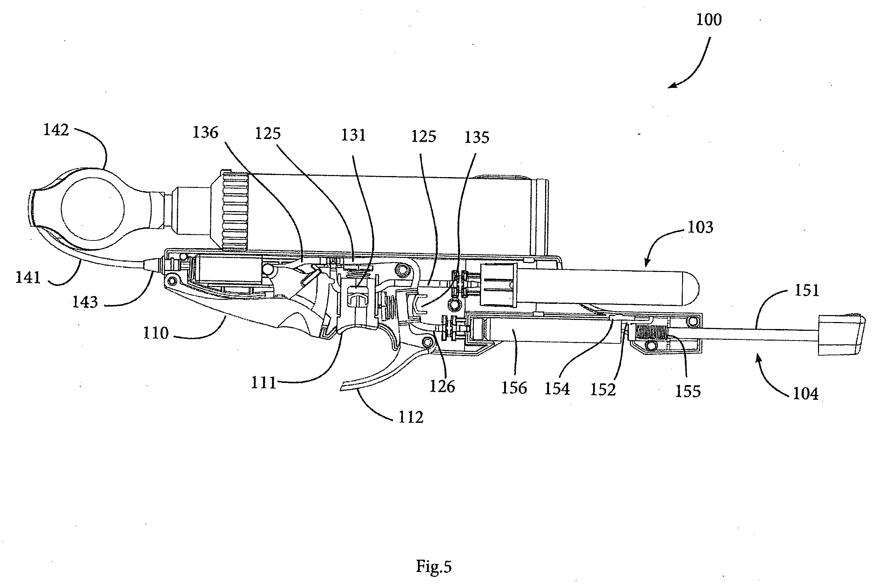

The drug delivery device 104 comprises the plunger 151 which presses against a ridge 152 on a latch 154 until a drug cartridge 156 is inserted. When the cartridge 156 is inserted it presses against the latch 154, causing the plunger 151 to move to press against the cartridge under action of a helical spring 155. The plunger 151 pushes on an internal plunger within the cartridge 156 which pressurises the medicinal compound. Fig. 5 shows the cartridge 156 in place and the plunger 151 about to move to the left to press against it.

The lumen 140 is configured to puncture tissue such as the tympanic membrane ("ear drum"), with excellent control because it extends from the spring- loaded mechanical extension mechanism 115 with the lever 110. This allows the user to extend the lumen 140 tip from within the speculum 142 when they are satisfied that they have identified the anterior inferior quadrant of the tympanic membrane using the otoscope 500 connected to the apparatus 100. The sheath 141 is trained into the speculum attachment 142 from which the lumen 140 can protrude. A lumen channel on the speculum attachment 142 can be used as a location marker to ensure the user punctures the correct location of the tympanic membrane and may be skived. The apparatus 100 may have various speculum attachments for different sizes of patient anatomy and different medical visualisation devices. Alternatively, the apparatus may be provided with only one type of speculum, of a universal type for use with a range of typical otoscopes.

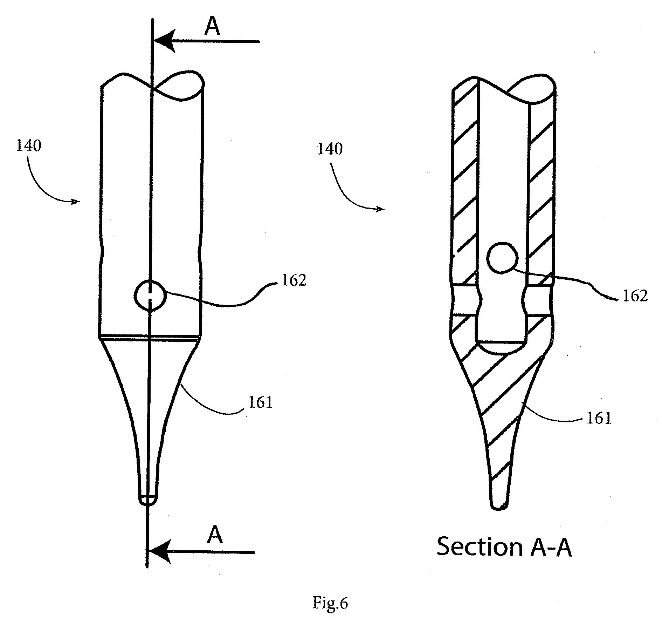

The lumen 140 will not in use penetrate skin or bone, but will penetrate the tympanic membrane. This is achieved as shown in Fig. 6 because it has a conical tip 161 which is solid with concave surfaces. There are two opposed apertures 162 for fluid flow. Due to its concave shape the conical tip 161 yields in use when it contacts skin or bone. The needle tip may be manufactured using a thermoforming process on a thermoplastic such as poly(ether-ether-ketone)-PEEK or similar. The needle tip 161 has a sufficiently sharp tip radius to penetrate the tympanic membrane at a force which can be imparted by the apparatus 100. The lumen channel will allow fluid to flow through the port holes 162. The needle tip 161, due to its concaved profile, forms a deformation or crumple zone immediately behind its point. If an attempt is made to penetrate a substrate which is more difficult to penetrate than the tympanic membrane, for example human or animal skin, the aforementioned zone would deform. The needle tip radius would sufficiently change during this deformation that it would prevent the needle penetrating the alternative substrate even if more force is applied to the needle.

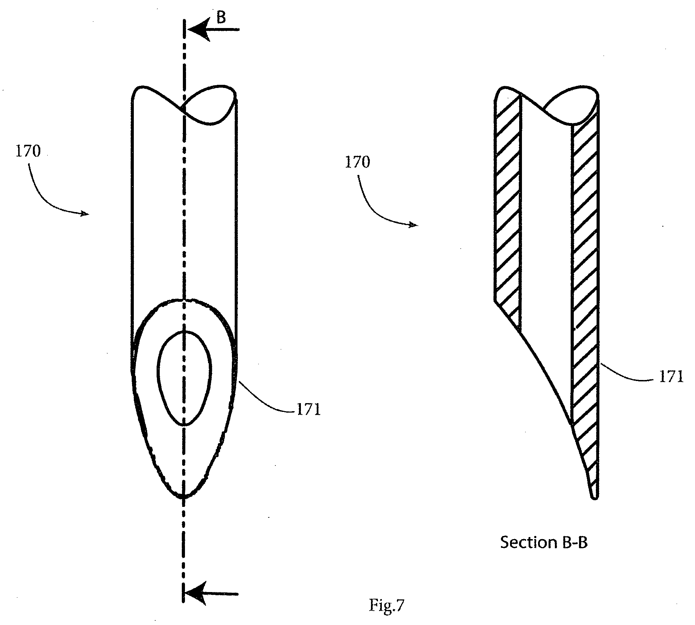

Referring again to Fig. 7, in an alternative embodiment a needle 170 is made from PEEK material or similar which has concave bevelled tip 171. This bevelled tip is machined in a single or numerous cutting directions from a standard cylindrical tube. The needle tip 171 has a sufficiently sharp point radius to penetrate the tympanic membrane at a force which can be imparted by the drug delivery device. The lumen channel will allow fluid to flow to and from the apparatus. The bevelled cut has a concave shape which would form a deformation or crumple zone immediately behind the needle point. If an attempt is made to penetrate a substrate which is more difficult to penetrate than the tympanic membrane, for example human or animal skin, the aforementioned zone would deform. The needle tip radius would sufficiently change during this deformation that it would prevent the needle penetrating the alternative substrate even if more force is applied to the needle.

The valve 111, 131 allows the user to activate the vacuum 103 to remove fluid from a space such as the middle ear. The default position for the (spring loaded) valve 111 is pressing against the flexible line 125 for the vacuum reservoir 103, pushing of the button 111 allowing vacuum reservoir 103 access to the junction 136 and hence to the lumen 140. The lever 112 allows the user to activate the pressurised drug supply 104 to administer medicinal product. The default position for the (spring loaded) valve handle 112 is against the flexible line 126 from the drug cartridge 156, pushing upwards of the lever 112 allowing drug supply to the lumen 140. As shown in Fig. 3, the flows are via puncture needles 158 and 157 which puncture the drug cartridge 156 and the vacuum cartridge 103 respectively. The medicinal product may comprise a suitable antibiotic in an amount that is effective to treat *otitis media.* Suitable anitbioitcs include those typically used for *otitis media* such as the quinolone antibiotics including, but not limited to, ofloxacin, difloxacin, enrofloxacin, ciprofloxacin, ibaflaxacin, marbofloxacin, and orbifloxacin and their pharmaceutically acceptable salts, e.g., the hydrochloride salt. For example, a 0.3wt% solution of ofloxacin may be used in an amount that is effective to treat *otitis media,* i.e. .2-10ml.

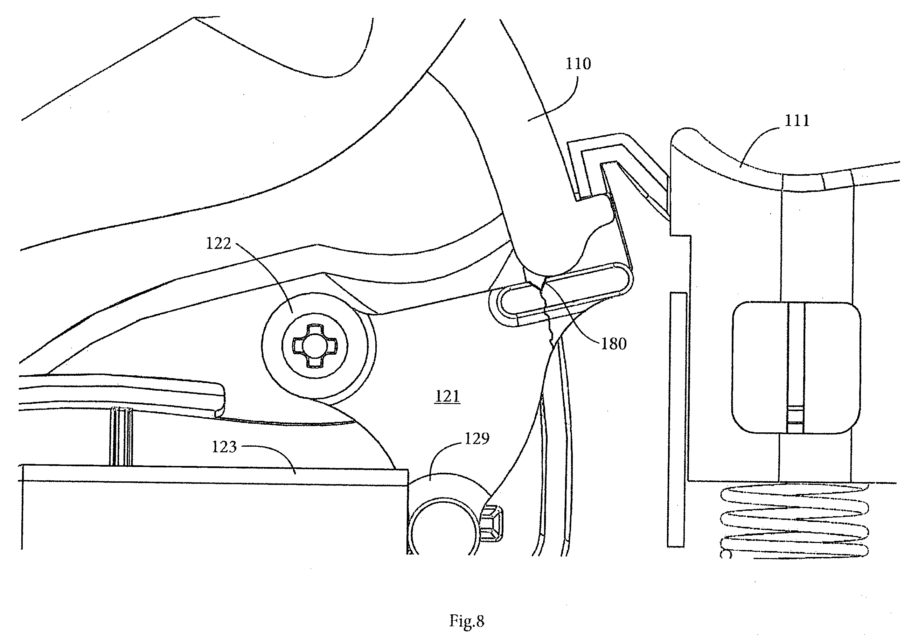

The apparatus 100 attaches to the third party otoscope 500 or some such visualisation device using the attachment clip 102 at the distal end of the apparatus. This embodiment allows for operation with one hand. The user supports the apparatus 100 using her thumb. The apparatus 100 allows the clinician to rest her small finger and ring finger on the patient's head while in use. This enables good stabilisation of the apparatus 100 and ensures that if there is any movement of the patient during operation the user will detect this movement and can retract the apparatus 100 without injuring the patient. The lever 110 is used to extend the lumen 140 by rotating the cam mechanism 121 about the pivot joint 122 of the cam on the casing 101. The cam 121 in turn linearly translates the rotary motion into translational motion of the carriage 123 which is attached to the needle 140. Fig. 8 shows this action in more detail. This diagram also shows that the lever 110 slides on the cam 121, but if excessive force is needed the cam 121 will break at a notch 180, thereby preventing further motion. This notch 180 would act as a failsafe if the user attempts to penetrate a substrate such as skin. The deformation of the needle tip would raise the force required to penetrate the skin. If the user continues to apply force to the lever 110 the notch 180 would cause the cam 121 to fail at the notch 180 and prevent imparting addition force to the lumen 140.

Importantly, the user's index finger is used for both the vacuum and drug delivery operations. This finger fits between the button 111 and the lever 112, and there is no space for other fingers.

Therefore it is not possible for the user to both activate the vacuum 103 and also drug delivery from the cartridge 156 at the same time.

The user installs the otoscope speculum 142 onto the head of the otoscope 500. The speculum 142 is sized depending on patient anatomy. The user then places the outer sheathing 141 into the speculum's channel. In other embodiments these may be pre-attached in the factory. The user will then install the vacuum cartridge 103. The puncture needle 157 will pierce the vacuum cartridge 103 to prime the system's vacuum line. The user then installs the drug cartridge 156. This is done by actuating the drug cartridge plunger 151, which compresses the drug cartridge compression spring 155.

Aided by the otoscope 500, the user locates the anterior inferior quadrant of the patient's tympanic membrane aided by the location marker of the needle lumen channel on the speculum, in some embodiments. Operation of the extension mechanism 115 extends the carriage 123. This in turn extends the lumen 140 which will perforate the tympanic membrane (ear drum). The extent of extension of the lumen 140 is also guided by depth or graduation marks on the lumen. Once the tympanic membrane is punctured, the user will press the vacuum push button 111 which will allow fluid to be aspirated from the middle ear through the lumen 140. The extent of vacuum and its duration can be controlled because the clinician can control the movement of the button 111 between the extreme positions. The user will then press the lever 112, to administer a prescribed dose of drug into the middle ear. Again, the extent of medicament flow and its duration can be controlled because the clinician can control the movement of the lever 112 between the extreme positions. The extension mechanism 115 is then released. Through the force exerted by the extension mechanism compression spring 124 the carriage 123 and the flexible lumen 140 return to their initial sheathed position. This can act as a safety mechanism for immediate retraction of the flexible lumen 140.

During the perforation of tissue such as the tympanic membrane, there is a high risk of damaging surrounding tissue. The lumen 140 is engineered such that it either buckles under the force required to damage any surrounding structures of the ear, and/or it deforms at the tip 160 as described above. The lumen 140 tip 160 however has sufficient axial strength and needle tip hardness to perforate the tympanic membrane.

Regarding buckling along its length, from Euler's buckling formula the buckling force:

$$F_{Buckling} = k\frac{EI}{L^2}$$

where $F_{Buckling}$ is the maximum force applied axially before buckling occurs, E is the Elastic/Young's Modulus of the material, I is the second moment of inertia (determined by the cross sectional area), L is the distance to the support point and k is a constant determined by the end conditions.

In summary, the following is the sequence of operation of the apparatus 100 for many applications.

- Remove apparatus from packaging.
- Attach correct speculum 142 onto the apparatus 100.
- Attach the apparatus 100 onto user's otoscope 500.
- Attach speculum 142 onto user's otoscope 500.
- Install vacuum cartridge 103 into the apparatus 100.
- Install drug cartridge 156 into the apparatus 100.
- Put the apparatus 100 into patient's ear, locate the anterior inferior quadrant of the tympanic membrane.
- Press the lever 110 to extend the lumen needle 140, which punctures the tympanic membrane.
- Press the vacuum push button 111 to aspirate fluid from the middle ear.
- Release the vacuum button 111 when complete.
- Push up the lever 112 using the same (index) finger to administer drug into the middle ear.
- Release the lever 112 when complete.
- Release the lever 110 to remove the needle lumen 140 from the middle ear.
- Remove the apparatus 100 from the vicinity of the patient's ear.

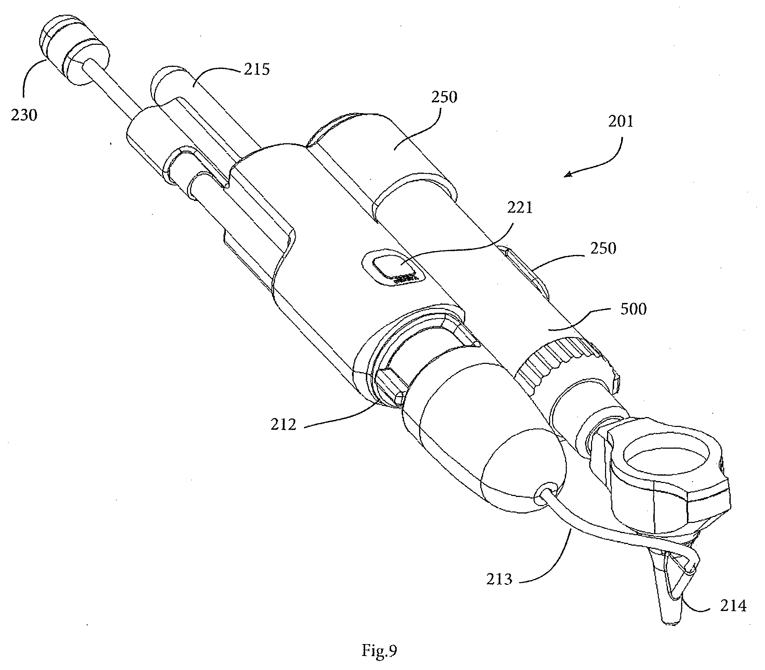

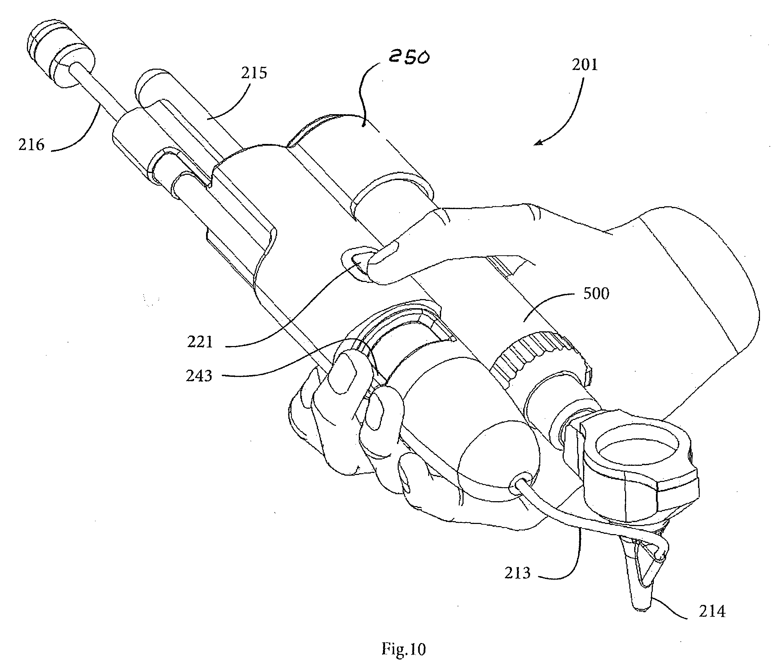

Referring to Figs. 9 to 15 an alternative apparatus 201 is illustrated. An otoscope 500 may be clipped to the apparatus 201 using a pair of opposed clips 250. An extension mechanism 212 extends and retracts the lumen 225 in a sheath 213. There is a vacuum reservoir 215, and a drug supply 216. These are linked with the extension mechanism 212 via a valve mechanism 220 having push-buttons 221 and 222. The push buttons have concave and convex heads to aid the user in distinguishing between the vacuum valve and drug delivery valve. There may be indicia marked on them also. The sheath 213 is trained into a speculum attachment 214 from which the lumen 225 can protrude. The needle lumen channel on the speculum attachment 214 can be used as a location marker to ensure the user punctures the correct location of the tympanic membrane and may be skived.

The drug reservoir 216 has a plunger 230, a cartridge compression spring 231, a drug cartridge housing 231a, a drug plunger spring 232, a puncture needle 233, and a fluid supply lumen 234.

The extension mechanism 212 comprises a housing 240, a compression spring 241, a traveller 242, and a turn wheel 243. The latter is threadably engaged with the housing so that rotation translates into axial movement to push the carriage 242. The flexible tubing 224 connects to the extension mechanism traveller 242 which in turn connects to the lumen 225. Flexible tubing 234 and 261 at the distal end of the Y-junction valve assembly 220 connects to the drug supply 216 and the vacuum reservoir 215 respectively. The main body of the Y-junction valve assembly 220 has two large holes to accommodate the opposed push buttons 221 and 222 and two small holes to accommodate a pinch pin. The push buttons 221 and 222 are spring loaded by compression springs. A Y-junction joins the drug fluid line 234 and the vacuum fluid line 261 to the flexible tubing 224 that is coupled with the carriage 242.

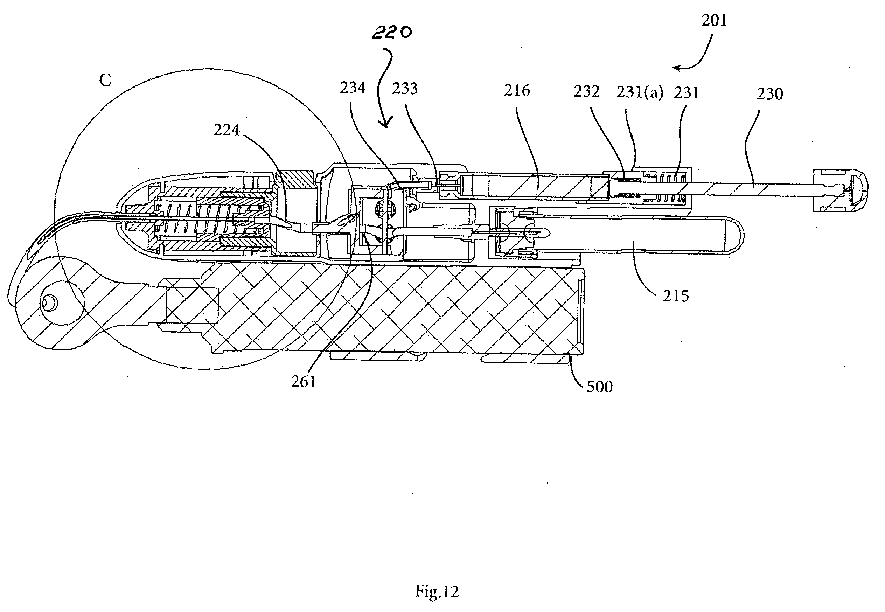

Referring particularly to Fig. 12, default pinching of the vacuum flexible tubing 261 prevents flow through the vacuum fluid line. The vacuum push button 221 collapses the vacuum flexible tubing 261 to prevent flow through it. A compression spring ensures that the valve is normally closed and flow only occurs when the vacuum push button 221 is actuated. The flow through the vacuum flexible tubing 261 can be regulated by varying the distance of actuation of the vacuum push button 221. The drug fluid line 234 operates in the same manner. If either the vacuum push button 221 or the drug push button 222 are actuated alone, the opposing valve remains closed. This mechanism prevents cross contamination of the drug and vacuum lines when using this apparatus. A break-away tab or ratchet mechanism may be present on the Y-Junction valve assembly 220 to prevent the user actuating the drug push button 222 prior to actuating the vacuum push button 221. This consists in one embodiment of a break away tab or pivoting pawl which would engage sloping teeth on the drug push button 222, thus preventing actuation. Once the vacuum push button 221 is actuated it would release the break-away or pivoting pawl to allow actuation of the drug push button 222. This prevents accidental administration of medicinal product prior to aspiration.

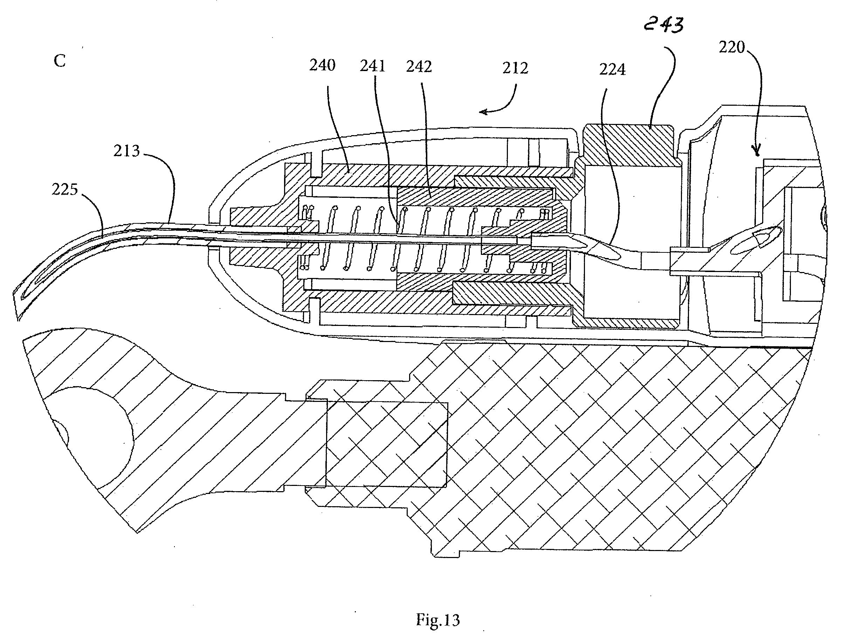

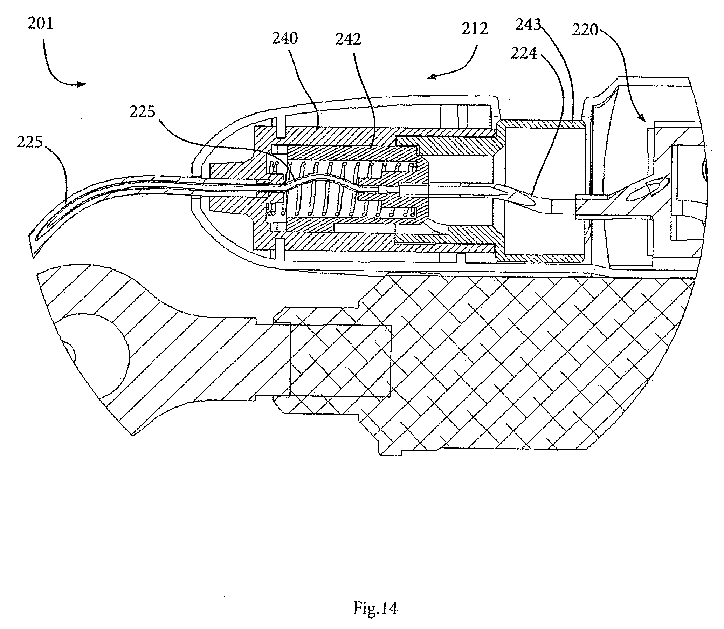

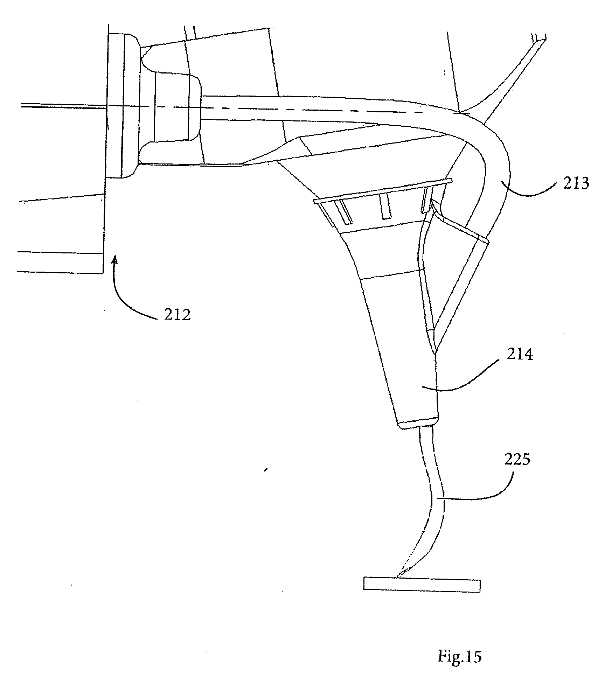

The extension mechanism traveller 242 and the lumen 225 are shown in the retracted and extended positions in Figs. 13 and 14 respectively. Fig. 14 shows that the axial force experienced by the lumen 225 has exceeded its buckling force, as given by the equation above, the lumen 225 being in a buckled state within the extension mechanism 212. Fig. 15 shows the alternative scenario, also governed by the above equation, in which buckling occurs closer to the tip of the lumen 225 due to excessive axial force through the needle.

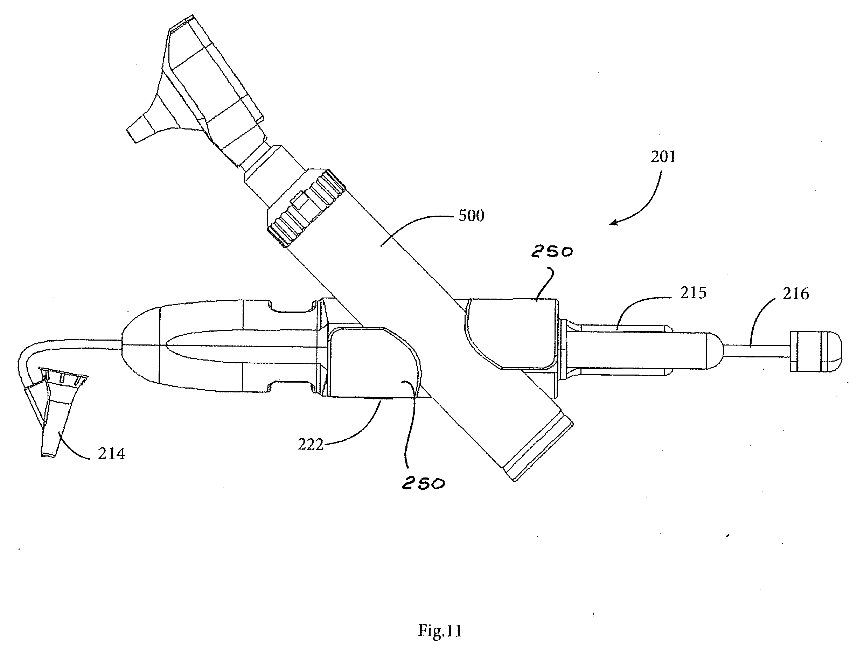

The user installs the otoscope speculum 214 onto the head of the otoscope 500, as best shown in Fig. 11. The speculum 214 is sized depending on patient anatomy. The user then places the flexible needle lumen 225 and outer sheathing 213 into the speculum's channel. The user will then install the vacuum cartridge 215. The vacuum cartridge needle assembly 260 will pierce the vacuum cartridge 215 to prime the system's vacuum line. The user then installs the drug cartridge 216. This is done by actuating the drug cartridge plunger 230, which compresses the drug cartridge compression spring 231, drug cartridge plunger compression spring 232 and drug cartridge housing 231 a. The drug cartridge 216 is installed by seating it into the apparatus 201. The user releases the cartridge plunger 230 which allows the drug cartridge installation housing 231a to push the drug cartridge 216 onto the drug cartridge needle assembly 233 which pierces the drug cartridge seal. The drug cartridge plunger 230 also actuates the plunger within the drug cartridge 216 which primes the drug line of the apparatus.

Aided by the otoscope 500, the user locates the anterior inferior quadrant of the patient's tympanic membrane aided by the location marker of the needle lumen channel on the speculum, in some embodiments. Operation of the extension mechanism 212 extends the component traveller 242. This in turn extends the flexible lumen needle 225 which will perforate the tympanic membrane (ear drum). Once the tympanic membrane is punctured, the user will press the vacuum push button 221 which will allow fluid to be aspirated from the middle ear through the flexible lumen needle 225. The user will then press the drug push button 222, to administer a prescribed dose of drug into the middle ear. The extension mechanism 212 is then released. Through the force exerted by the extension mechanism compression spring 241 the component traveller 242 and the flexible lumen 225 return to their initial sheathed position. This can act as a safety mechanism for immediate retraction of the flexible lumen needle 225.

It will be appreciated from the above two embodiments that the apparatus allows simple single-handed operation with the user being able to control in a safe and controlled manner application of a vacuum and the drug delivery. This is performed through a lumen which may deform at its tip or which may buckle somewhere along it length proximally from the tip if it encounters an obstruction which it is not desired to puncture, such as the malleus, external ear canal.

The invention is not limited to the embodiments described but may be varied in construction and detail. For example, the apparatus may attach to or be used with other visualisation devices, for example an endoscope, a videoscope, a microscope, or an ophthalmoscope. The apparatus can be used for other medical procedures which involve either removal of fluid from the body and/or administration of fluid into the body. This could include but is not limited to tympanocentesis, drug/biological compound delivery, and saline flushing. The speculum attachment may be attached by the user or in another embodiment the apparatus comes pre-assembled with the speculum attached to the needle lumen sheath. In another embodiment, the needle lumen sheath will stop at a distance from the tip of the speculum. This would improve the user's field of vision through the otoscope. The channel would be angled to promote skiving of the lumen needle. This ensures that the puncture site is within the field of view of the user. The extension mechanism may be actuated using alternative methods to those illustrated. These could include, but are not limited to, an extension lever, an extension push button, an extension slider, or an extension scroll wheel. In some embodiments the tip of the lumen is manufactured from an inflexible material such as stainless steel, or titanium. In some embodiments, the extension mechanism may have a feature which biases the lumen to buckle in a particular direction. Similarly, the lumen may have a feature in-built which promotes buckling in a particular direction within the extension mechanism. These embodiments may lower the force required to buckle/collapse the lumen needle. Also, the vacuum and supply valves may be integrated into a single valve device which has ports receiving both the vacuum and supply ports.

**Claims**

1. An apparatus (100) for removal and delivery of fluids to a human or animal body, the apparatus comprising:

    a housing (101),
    a lumen (140) adapted to extend from the housing;
    a vacuum device (103) for application of negative pressure in the lumen;
    a fluid supply (104) for delivery of a medicament in the lumen;
    a vacuum valve (111, 131) between the vacuum device (103) and the lumen, and a supply valve (112, 135) between the fluid supply and the lumen;
    user actuators (111, 112) for control of the valves; and
    an extension mechanism (110,115) for controlling extension and retraction of the lumen with respect to the apparatus housing,
    **characterized in that**,
    the actuators (111, 112) are arranged so that a single finger can be used to operate both actuators, in which the actuators are aligned whereby inward finger movement operates one valve (111) and outward movement of the same finger operates another valve (112).

2. An apparatus as claimed in claim 1, wherein the fluid supply (156) and the vacuum device (103) extend from a distal end of the housing.

3. An apparatus as claimed in any preceding claim, wherein the fluid supply includes a plunger (151) in a reservoir (156) having an outlet linked with the valve, and wherein the vacuum device (103) comprises a sealed vacuum chamber which may be pierced, and wherein the valves are linked in a Y configuration (136) with a single outlet to the lumen

4. An apparatus as claimed in any preceding claim, wherein at least one valve is adapted to allow intermediate positions between fully open and fully closed, and a default closed position, and wherein there is infinite variability between said open and closed positions, and wherein at least one valve is adapted to remain open for a variable duration according to user operation of the actuator, and wherein at least one valve includes a spring-loaded pinching member (111) to pinch a flexible tube (125), and wherein the pinching member is biased to press the flexible conduit against a static part (131) of the apparatus.

5. An apparatus as claimed in any preceding claim, wherein the apparatus further comprises a safety feature (221, 222) which prevents fluid delivery before application of a vacuum.

6. An apparatus as claimed in any preceding claim, wherein one actuator comprises a push button (111) and the other actuator comprises a lever (112).

7. An apparatus as claimed in any preceding claim, wherein the extension mechanism comprises a spring-biased carriage arranged to slide under user control with an actuator (110) to push the lumen (141), and to allow it to retract, and wherein the carriage (123) is adapted to automatically retract under spring bias to retract the lumen.

8. An apparatus as claimed in any of claims 1 to 7, wherein the extension mechanism actuator comprises a rotatable member (121) threadably engaged with the housing to move axially upon user rotation.

9. An apparatus as claimed in any preceding claim, wherein the lumen is adapted to deform or bend (140) if an obstruction is encountered in the ear area which is not the tympanic membrane, and wherein the lumen has a flexibility causing it to deform by buckling, and wherein the lumen has a tip (140) which is configured to deform, and wherein the tip has a concave surface, and wherein the tip has a conical shape.

10. An apparatus as claimed in claim 9, wherein the tip (161) is solid, without a channel, and a lumen channel terminates in one or more apertures (162) near the tip.

11. An apparatus as claimed in claims 9 or 10, wherein the lumen tip (161) narrows with a bevelled concave surface on one side.

12. An apparatus as claimed in any preceding claim, wherein the extension mechanism (220) comprises a lever actuator for causing movement of the lumen, and wherein the actuator (180) is adapted to yield if excessive force is applied to move a lumen into an obstruction.

**13.** An apparatus as claimed in any preceding claim, wherein the apparatus comprises a fixture (102) to connect with a visualisation device such as an otoscope (500), and wherein the fixtures are arranged to retain the visualisation device in an orientation parallel to the housing, and wherein the apparatus further comprises a speculum (214) linked by a sleeve to the housing, the lumen extending through the sleeve.

**14.** An apparatus as claimed in claim 13, wherein the speculum is adapted to be connected to a visualisation device such as an otoscope (500).

**15.** An apparatus as claimed in claims 13 or 14, wherein the speculum (214) comprises a channel to receive and retain the sleeve, and wherein the speculum is configured to assist location of the lumen for a desired application of the apparatus.

**Patentansprüche**

**1.** Vorrichtung (100) zum Absaugen und Zuführen von Fluiden zu einem menschlichen oder tierischen Körper, wobei die Vorrichtung Folgendes umfasst:

ein Gehäuse (101),
ein Lumen (140), das dazu angepasst ist, sich von dem Gehäuse zu erstrecken;
eine Vakuumeinrichtung (103) zum Anlegen von Unterdruck in dem Lumen;
eine Fluidversorgung (104) zum Zuführen eines Medikaments in dem Lumen;
ein Vakuumventil (111, 131) zwischen der Vakuumeinrichtung (103) und dem Lumen, und ein Versorgungsventil (112, 135) zwischen der Fluidversorgung und dem Lumen;
Benutzerbetätigungselemente (111, 112) zum Steuern der Ventile; und
einen Ausfahrmechanismus (110, 115) zum Steuern des Ausfahrens und Einfahren des Lumens in Bezug auf das Vorrichtungsgehäuse, **dadurch gekennzeichnet, dass**
die Betätigungselemente (111, 112) derart angeordnet sind, dass ein einziger Finger zum Betätigen beider Betätigungselemente verwendet werden kann, wobei die
Betätigungselemente miteinander ausgerichtet sind, wodurch eine Fingerbewegung nach innen ein Ventil (111) betätigt und eine Bewegung nach außen desselben Fingers ein anderes Ventil (112) betätigt.

**2.** Vorrichtung nach Anspruch 1, wobei sich die Fluidversorgung (156) und die Vakuumeinrichtung (103) von einem distalen Ende des Gehäuses erstrecken.

**3.** Vorrichtung nach einem der vorangehenden Ansprüche, wobei die Fluidversorgung einen Kolben (151) in einem Behälter (156), der einen mit dem Ventil verbundenen Auslass aufweist, umfasst, und wobei die Vakuumeinrichtung (103) eine verschlossene Vakuumkammer umfasst, die durchstochen werden kann, und wobei die Ventile in einer Y-Konfiguration (136) mit einem einzigen Auslass zu dem Lumen verbunden sind.

**4.** Vorrichtung nach einem der vorangehenden Ansprüche, wobei mindestens ein Ventil dazu angepasst ist, Zwischenstellungen zwischen ganz offen und ganz geschlossen und eine geschlossene Standardstellung zuzulassen, und wobei zwischen der offenen und der geschlossenen Stellung unendliche Verstellbarkeit besteht, und wobei mindestens ein Ventil dazu angepasst ist, für eine veränderliche Dauer gemäß Benutzerbetätigung des Betätigungselements offen zu bleiben, und wobei mindestens ein Ventil ein federbelastetes Quetschglied (111) zum Quetschen einer flexiblen Röhre (125) umfasst und wobei das Quetschglied vorgespannt ist, um die flexible Leitung an einen unbeweglichen Teil (131) der Vorrichtung zu drücken.

**5.** Vorrichtung nach einem der vorangehenden Ansprüche, wobei die Vorrichtung weiter ein Sicherheitsmerkmal (221, 222) umfasst, das die Fluidzufuhr vor dem Anlegen eines Vakuums verhindert.

**6.** Vorrichtung nach einem der vorangehenden Ansprüche, wobei ein Betätigungselement einen Drucktaster (111) umfasst und das andere Betätigungselement einen Hebel (112) umfasst.

**7.** Vorrichtung nach einem der vorangehenden Ansprüche, wobei der Ausfahrmechanismus einen federgespannten Schlitten umfasst, der dazu angeordnet ist, unter Benutzersteuerung mit einem Betätigungselement (110) zu gleiten, um das Lumen (141) zu schieben und es einfahren zu lassen, und wobei der Schlitten (123) dazu angeordnet ist, unter Federspannung automatisch einzufahren, um das Lumen einzufahren.

**8.** Vorrichtung nach einem der Ansprüche 1 bis 7, wobei das Ausfahrmechanismus-Betätigungselement ein drehbares Glied (121) umfasst, das sich in Gewindeeingriff mit dem Gehäuse befindet, um sich bei Drehung durch den Benutzer axial zu bewegen.

**9.** Vorrichtung nach einem der vorangehenden Ansprüche, wobei das Lumen dazu angepasst ist, sich zu verformen oder zu biegen (140), wenn ein Hindernis im Ohrbereich angetroffen wird, bei dem es sich nicht um das Trommelfell handelt, und wobei das Lumen eine Flexibilität aufweist, die es veranlasst, sich durch Knicken zu verformen, und wobei das Lumen eine Spitze (140) aufweist, die dazu konfiguriert ist, sich zu verformen, und wobei die Spitze eine konkave Oberfläche aufweist, und wobei die Spitze eine Kegelform aufweist.

10. Vorrichtung nach Anspruch 9, wobei die Spitze (161) massiv, ohne einen Durchgang ist und ein Lumendurchgang in einer oder mehreren Öffnungen (162) in der Nähe der Spitze endet.

11. Vorrichtung nach Anspruch 9 oder 10, wobei die Lumenspitze (161) mit einer abgeschrägten konkaven Oberfläche auf einer Seite schmaler wird.

12. Vorrichtung nach einem der vorangehenden Ansprüche, wobei der Ausfahrmechanismus (220) ein Hebelbetätigungselement zum Bewirken der Bewegung des Lumens umfasst, und wobei das Betätigungselement (180) dazu angepasst ist, nachzugeben, wenn übermäßige Kraft ausgeübt wird, um ein Lumen in ein Hindernis zu bewegen.

13. Vorrichtung nach einem der vorangehenden Ansprüche, wobei die Vorrichtung eine Halterung (102) zum Koppeln mit einer Visualisierungsvorrichtung, wie etwa einem Otoskop (500), umfasst, und wobei die Halterungen dazu angeordnet sind, die Visualisierungsvorrichtung in einer zum Gehäuse parallelen Orientierung zu halten, und wobei die Vorrichtung weiter ein durch eine Hülse mit dem Gehäuse verbundenes Spekulum (214) umfasst, wobei sich das Lumen durch die Hülse erstreckt.

14. Vorrichtung nach Anspruch 13, wobei das Spekulum dazu angepasst ist, mit einer Visualisierungsvorrichtung, wie etwa einem Otoskop (500), gekoppelt zu werden,

15. Vorrichtung nach Anspruch 13 oder 14, wobei das Spekulum (214) einen Durchgang umfasst, um die Hülse aufzunehmen und zu halten, und wobei das Spekulum dazu konfiguriert ist, das Fixieren des Lumens für eine gewünschte Anwendung der Vorrichtung zu unterstützen.

**Revendications**

1. Appareil (100) à des fins de retrait et de distribution de fluides dans le corps d'un être humain ou d'un animal, l'appareil comportant :

un boîtier (101),
une lumière (140) adaptée à des fins d'extension en provenance du boîtier ;
un dispositif de vide (103) à des fins d'application d'une pression négative dans la lumière;

une alimentation en fluide (104) à des fins de distribution d'un médicament dans la lumière;
une valve de vide (111, 1131) entre le dispositif de vide (103) et la lumière, et une valve d'alimentation (112, 135) entre l'alimentation en fluide et la lumière;
des actionneurs d'utilisateur (111, 112) à des fins de commande des valves; et
un mécanisme d'extension (110, 115) à des fins de commande de l'extension et
de la rétraction de la lumière par rapport au boîtier de l'appareil,
**caractérisé en ce que**,
les actionneurs (111, 112) sont agencés de telle sorte qu'un seul doigt peut être utilisé pour actionner les deux actionneurs, les actionneurs étant alignés ce par quoi un mouvement du doigt vers l'intérieur actionne une valve (111) et un mouvement vers l'extérieur du même doigt actionne une autre valve (112).

2. Appareil selon la revendication 1, dans lequel l'alimentation en fluide (156) et le dispositif de vide (103) s'étendent depuis une extrémité distale du boîtier.

3. Appareil selon l'une quelconque des revendications précédentes, dans lequel l'alimentation en fluide comprend un piston plongeur (151) dans un réservoir (156) ayant une sortie reliée à la valve, et dans lequel le dispositif de vide (103) comporte une chambre de vide scellée qui peut être percée, et dans lequel les valves sont reliées selon une configuration en Y (136) avec une seule sortie à la lumière.

4. Appareil selon l'une quelconque des revendications précédentes, dans lequel au moins une valve est adaptée pour permettre des positions intermédiaires entre la position entièrement ouverte et la position entièrement fermée et une position fermée par défaut, et dans lequel il y a une variabilité infinie entre lesdites positions ouverte et fermée, et dans lequel au moins une valve est adaptée pour rester ouverte pendant une durée variable en fonction de l'actionnement par l'utilisateur de l'actionneur, et dans lequel au moins une valve comprend un élément de pincement à ressort (111) servant à pincer un tube flexible (125), et dans lequel l'élément de pincement est sollicité à des fins de compression du conduit flexible contre une partie statique (131) de l'appareil.

5. Appareil selon l'une quelconque des revendications précédentes, dans lequel l'appareil comporte par ailleurs un organe de sécurité (221, 222) qui empêche la distribution du fluide avant l'application d'un vide.

6. Appareil selon l'une quelconque des revendications précédentes, dans lequel un actionneur comporte un bouton-poussoir (111) et l'autre actionneur comporte un levier (112).

7. Appareil selon l'une quelconque des revendications précédentes, dans lequel le mécanisme d'extension comporte un chariot à ressort agencé à des fins de coulissement sous la commande de l'utilisateur avec un actionneur (110) pour pousser la lumière (141), et pour en permettre la rétraction, et dans lequel le chariot (123) est adapté à des fins de rétraction automatique sous l'effet d'une sollicitation du ressort pour rétracter la lumière.

8. Appareil selon l'une quelconque des revendications 1 à 7, dans lequel l'actionneur du mécanisme d'extension comporte un élément rotatif (121) mis en prise par filetage avec le boîtier à des fins de mouvement dans le sens axial lors de la rotation par l'utilisateur.

9. Appareil selon l'une quelconque des revendications précédentes, dans lequel la lumière est adaptée pour se déformer ou se plier (140) si une obstruction est rencontrée dans la partie de l'oreille qui n'est pas la membrane du tympan, et dans lequel la lumière a une flexibilité lui permettant de se déformer par distorsion, et dans lequel la lumière a un embout (140) qui est configuré à des fins de déformation, et dans lequel l'embout a une surface concave, et dans lequel l'embout a une forme conique.

10. Appareil selon la revendication 9, dans lequel l'embout (161) est solide, sans canal, et un canal de lumière se termine dans une ou plusieurs ouvertures (162) à proximité de l'embout.

11. Appareil selon la revendication 9 ou la revendication 10, dans lequel l'embout de lumière (161) se rétrécit avec une surface concave biseautée d'un côté.

12. Appareil selon l'une quelconque des revendications précédentes, dans lequel le mécanisme d'extension (220) comporte un actionneur de levier servant à entraîner le mouvement de la lumière, et dans lequel l'actionneur (180) est adapté pour céder si une force excessive est appliquée à des fins de mouvement d'une lumière dans une obstruction.

13. Appareil selon l'une quelconque des revendications précédentes, dans lequel l'appareil comporte un agencement (102) à des fins de connexion à un dispositif de visualisation tel un otoscope (500), et dans lequel les agencements sont agencés à des fins de retenue du dispositif de visualisation selon une orientation parallèle par rapport au boîtier, et dans lequel l'appareil comporte par ailleurs un spéculum (214) relié par un manchon au boîtier, la lumière s'étendant au travers du manchon.

14. Appareil selon la revendication 13, dans lequel le spéculum est adapté à des fins de connexion à un dispositif de visualisation tel un otoscope (500).

15. Appareil selon la revendication 13 ou la revendication 14, dans lequel le spéculum (214) comporte un canal pour recevoir et retenir le manchon, et dans lequel le spéculum est configuré pour faciliter la mise en position de la lumière pour une application souhaitée de l'appareil.

Fig.1

Fig.2

Fig.3

Fig.4

Fig.5

Section A-A

Fig.6

Fig.7

Section B-B

171

170

171

170

B

Fig.8

Fig.9

Fig.10

Fig.11

Fig.12

EP 2 709 685 B1

Fig.13

EP 2 709 685 B1

EP 2 709 685 B1

Fig.14

Fig.15

213

214

225

212

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6024726 A
- US 5664094 A
- US 6390975 B
- WO 200511552 A
- US 5902264 A, Toso
- EP 1389458 A